# EUROPEAN PATENT SPECIFICATION

(11) **EP 0 751 227 B1**
(45) Date of publication and mention of the grant of the patent: **14.07.2004**
(21) Application number: 96303105.9
(22) Date of filing: 02.05.1996
(51) Int. Cl.: C12Q 1/68, C12P 19/34, C07H 21/04

(54) **Detection and identification of fungi**
Nachweis und Identifizierung von Pilzen
Détection et identification de champignons

(30) Priority: 04.05.1995 GB 9509112
(43) Date of publication of application: 02.01.1997
(73) Proprietor: SCOTTISH CROP RESEARCH INSTITUTE, Dundee DD2 5DA Scotland (GB)
(72) Inventor: Dolan, Alison, Monifieth, Dundee, DD5 4RP (GB); Duncan, James Moffat, Dundee, DD2 1SL (GB); Cooke, David Edward Llewelyn, Dundee, DD1 5NY (GB)
(74) Representative: Pattullo, Norman

(56) References cited:
- WO-A-93/17117
- WO-A-94/13832
- LEE S B ET AL: "PHYLOGENY OF FIVE FUNGUS-LIKE PROTOCTISTAN PHYTOPHTHORA -SPP INFERRED FROM THE INTERNAL TRANSCRIBED SPACERS OF RIBOSOMAL DNA." MOL BIOL EVOL 9 (4). 1992. 636-653. CODEN: MBEVEO ISSN: 0737-4038, XP002043599
- WHITE T ET AL: "AMPLIFICATION AND DIRECT SEQUENCING OF FUNGAL RIBOSOMAL RNA GENES FOR PHYLOGENETICS" PCR PROTOCOLS, GUIDE TO METHODS AND APPLICATIONS, 1990, INNIS M A;GELFAND D H; SNINSKY J J; WHITE T J, pages 315-322, XP002017490
- FALKENSTEIN K F ET AL: "DIFFERENTIATION OF GROUP IV PHYTOPHTHORA SPECIES BY PCR AMPLIFICATION OF NUCLEAR RIBOSOMAL DNA INTERNAL TRANSCRIBED SPACER REGION 2." ANNUAL MEETING OF THE AMERICAN PHYTOPATHOLOGICAL SOCIETY, ST. LOUIS, MISSOURI, USA, AUGUST 17-21, 1991. PHYTOPATHOLOGY 81 (10). 1991. 1157. CODEN: PHYTAJ ISSN: 0031-949X, XP002043600
- COOKE D E L ET AL: "Phylogenetic analysis of Phytophthora species based on ITS1 and ITS2 sequences of the ribosomal RNA gene repeat." MYCOLOGICAL RESEARCH 101 (6). 1997. 667-677. ISSN: 0953-7562, XP002043601

## Description

This invention relates to a method for the detection and identification of fungi in eg culture, plant material, soil, propagation media or water using oligonucleotide primers to amplify (using the polymerase chain reaction) or hybridise to fungal nucleic acid, and to polynucleotides such as primers for use in the method.

The Oomycetes class contains some of the most important and damaging fungal pathogens of plants, especially within the genera *Phytophthora*, *Pythium* and *Peronospora* which are regarded as members of the order *Peronosporales*. *Phytophthora* comprises around sixty species, all of them pathogenic on plants; some highly specialised on one or a few hosts, others with a very wide host range of several thousands of plants. A list of the ten most commercially significant diseases worldwide might include late blight of potato, caused by *Phytophthora infestans* and black pod of cocoa caused by several *Phytophthora* spp. and various forms of disease on eucalypts caused by *P. cinnamomi. Pythium* is also important; it is a much more diverse group of generally unspecialised fungi, mostly causing damping-off diseases of seedlings and succulent plant tissues. *Peronospora* is typical of the biotrophic and obligate downy mildews, highly specialised pathogens of the leaves and stems of plants. There are several genera and many species of downy mildew, each generally with a restricted host range.

The present invention provides a method of identifying a fungi at the genera level and at the species level using the Polymerase Chain Reaction (PCR). Briefly, by PCR, a target DNA sequence is amplified exponentially to levels detectable by non-radioactive techniques. Two short pieces of DNA (primers) usually between 10 and 30 nucleotides long are synthesised by standard chemical methods. The DNA sequence of the 'forward' primer complements the sequences at the start of the target DNA in the downstream strand of DNA in the double DNA helix, while the 'reverse' primer sequence complements the end of the target sequence in the complementary DNA strand.

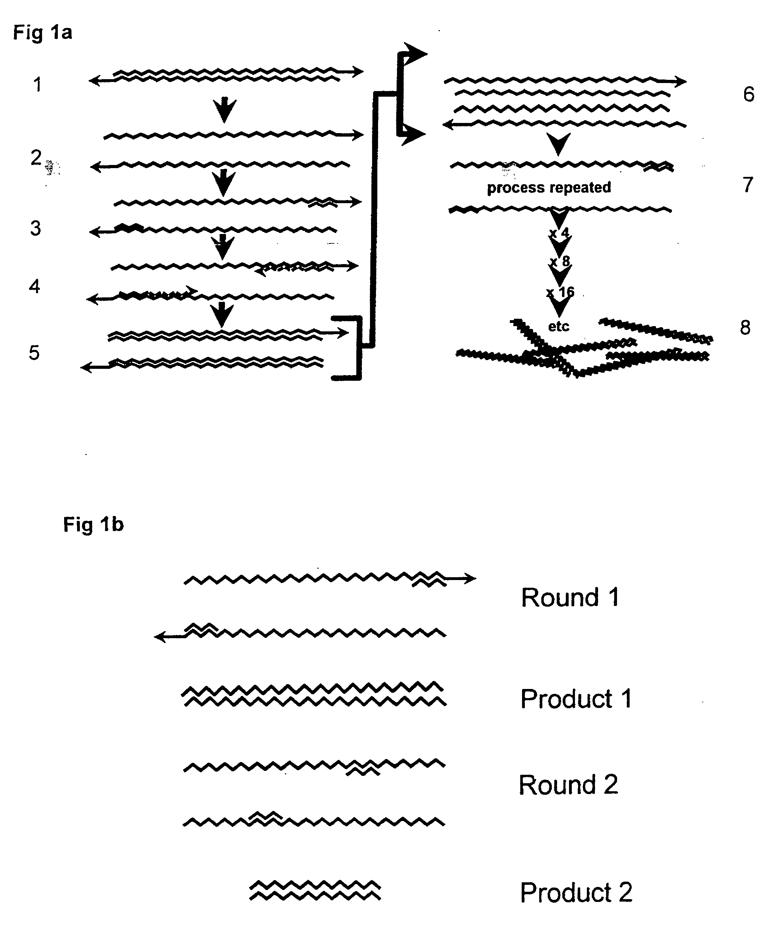

The primers are added to the test DNA along with the enzyme *Taq* polymerase, a mixture of the four deoxyribonucleotides found in DNA, and buffers etc. The mixture is then heated to a temperature (Figure 1) at which the two strands of the target DNA separate. On cooling, the primers, which are in excess in the reaction mixture, attach to complementary sequences on the separated single strands (annealing), and the *Taq* polymerase then synthesises new double strands of DNA starting at the ends of the short helices formed by the annealing of the primers to the double strands. A new strand is formed in the forward sense from the forward primer and in the reverse sense from the reverse primer. *Taq* polymerase is not destroyed at high temperatures and will work through repeated cycles of the reaction. Thus the whole reaction can be stopped by raising the temperature again until the strands separate and then repeated by lowering the temperature to allow annealing and DNA synthesis.

After the first temperature cycle, four strands function as templates but two very short strands representing only the sequence between the primers are synthesised. These short strands are doubled in number with each cycle whereas the original long strands are not. Within 35 reaction cycles, a few copies of the target sequence can be amplified into millions of copies. As these are of uniform length, they can be concentrated into a single band by electrophoresis, and their presence can be revealed by staining with ethidum bromide or other reagents. Thus, a DNA sequence that is unique to a particular organism can be amplified to detectable levels against a background of other DNA if appropriate primers can be made for that unique DNA sequence.

Nested PCR (a variant of the PCR technique) requires two sets of primers. The first set produces an amplification product as described above. The second set of primers must be complementary to sequences within this product. By adding a small amount of the reaction product from the first reaction cycle to another reaction, a short DNA sequence internal to the original product can be amplified. Nested PCR has two advantages: the first set of primers can be less specific and thereby allow the amplification of DNA from several possible target organisms - specificity can then be achieved by using more specific primers in the second reaction, and it increases sensitivity with two rounds of amplification.

The invention particularly concerns primers complementary to rDNA. The ribosomal RNA gene repeat (rDNA) encodes RNA which after processing makes up the RNA subunits within ribosomes. The subunits are highly conserved across all organisms. Coding regions of the large and small subunit rDNA show high levels of sequence homology. The Internal Transcribed Spacer regions (ITS1 and ITS2) which lie between the 18S and 28S genes are transcribed but subsequently removed prior to ribosome assembly (Nues *et al.*, 1994).

According to the present invention there is provided a polynucleotide having a sequence corresponding to at least a part of any one of SEQ:ID:Nos 1-11, or a complementary strand thereof.

Further according to the invention there is provided the use of any one of such polynucleotides for the detection of a fungal species, particularly a *Peronospora,* and more particularly a *Phytophthora* species.

Further according to the invention there is provided a polynucleotide having a sequence corresponding to at least a part of any one of SEQ:ID:Nos 12-39, or a complementary strand thereof.

Further according to the invention there is provided the use of a polynucleotide as described above having a sequence complementary to at least a part of the species-specific sequence of ITS region nucleic acid, or ITS flanking region nucleic acid, for the detection of that species.

The invention includes both complementary strands of a double stranded polynucleotide sequence.

Preferably, the polynucleotide has a sequence corresponding to at least a part of any one of sequences SEQ:ID:Nos 1-39.

Preferably, the primers and polynucleotides of the invention can detect a particular species of *Peronosporales*, preferably a particular *Phytophthora* species, in a mixture of different species.

Preferably, the primers and polynucleotides of the invention are used in a nucleotide hybridisation assay such as a polymerase chain reaction assay to identify the species of *Phytophthora*.

Combinations of more than one primer and/or polynucleotide may be used in the same assay. For example, the assay may use a pair of primers (one forward, and one reverse) chosen from the above-listed polynucleotides. It is sufficient for only one of the polynucleotides (eg the forward primer) to complement a unique sequence in the target DNA to be amplified and detected, but it is preferred for both the forward and reverse primers to be complementary to unique sequences in the target DNA.

Further according to the present invention there is provided a method of detecting the presence of nucleic acid characteristic of a species of fungus, the method comprising providing a sample of material to be tested which is suspected of containing or consisting of nucleic acid from said species of fungus, providing a polynucleotide having a sequence complementary to at least a part of the species-specific sequence of ITS region DNA or ITS flanking region DNA of said species of fungus or an equivalent or a functional analogue thereof, exposing the polynucleotide to the material, and determining whether reaction between the polynucleotide and the sample of material has occurred.

The material sample can comprise plant tissue, animal tissue, or food inanimate tissue such as soil.

Preferably the reaction comprises binding of the polynucleotide and the material, and optionally involves amplification of DNA.

Preferably, the polynucleotide used in the method consists of, includes or is derived from any one of sequences SEQ:ID:Nos 1-39, and most preferably any one of SEQ:ID:Nos1-11.

The advantages of a PCR-based specific amplification based test over previously recorded methods are speed, simplicity and sensitivity. Crude preparations of fungal material provide sufficient DNA to allow identification. Mycelium, zoospores or oospores may be treated by sonication or freeze-thaw cycles to release sufficient DNA of a purity suitable for the PCR process. This removes the need to culture fungal mycelium and extract DNA using conventional time consuming methods. The PCR amplification process is considerably easier than the blotting of DNA onto nylon membranes and hybridisation with a radiolabelled probe. A PCR reaction can be set up and run within 3 hours. Sensitivity recorded to date has been in the region of 100 fg to 10 pg of pure DNA which is greater than that through hybridisation because PCR amplifies the DNA.

While further modifications and improvements may be made without departing from the scope of this invention, the following is a description of one or more examples of the invention, with reference to the accompanying drawings.

### Brief description of the Drawings:

Fig 1a is a schematic representation of the steps involved in the Polymerase Chain Reaction (PCR);
Fig 1b is a schematic representation of the steps involved in nested PCR;
Fig 2 is a negative image of an ethidium bromide-stained gel of PCR products amplified with *P.cambivora* specific primers (Primer 2 and Primer 3) with DNA of a range of *Phytophthora* species. Lane 1-9 *P.fragariae* var *fragariae* races A1, A2, A3, A4, A6, A7, A8, A9 and A10, Lane 10 *P.cambivora*, Lane 11 *P.cinnamomi*, Lane 12 *P.fragariae* var *rubi,* Lane 13 *P.cactorum,* Lane 14, *P.cryptogea*, Lane 15 *P.drechsleri,* Lane 16 *P.megasperma*, Lane 17 control. L DNA ladder;
Fig 3 shows the detection of *Phytophthora cryptogea* in infected tomato plants. 1 - marker lane; 2 - pure DNA from *P. cryptogea*; 3 - negative control without DNA; 4 - 9 infected tomato stems;

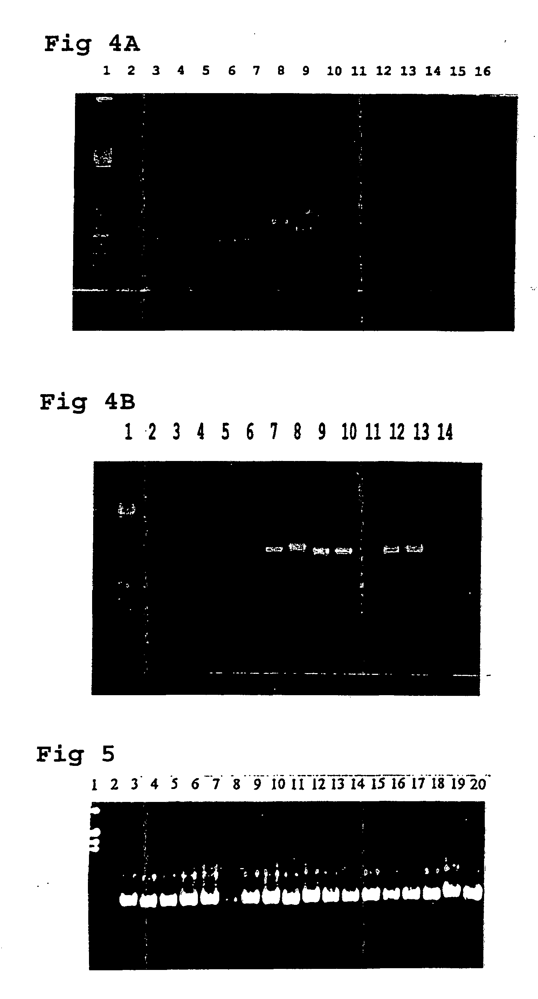

Fig 4A shows an agarose gel separating amplification products obtained with (A) the universal primer combination ITS6 and ITS4 and (B) the *Peronosporales* primer 8 and the universal primer ITS4. In A: Lanes 1-markers; lane 2 - *Phaeodactylum tricornata*; lane 3 - *Saprolegnia ferax*; lane 4 *- Thraustotheca clavata;* lane 5 *- Achyla radiosa*; lane 6 *- Saprolegnia turfosa*; lane 7 - *Brevilegnia gracilis*; lane 8 *- Pythium sylvaticum**; lane *9 - Phytophthora infestans**; lane 10 - *Peronospora viciae**; lanes 11 - to 14 - four isolates of *Peronospora sparsa**; lane 15 - *Albugo candida**; lane 16 - control, no DNA.
4B. Amplification products obtained with the primer combination DC6 and ITS4: Lanes 1- markers; lane 2 - *Phaeodactylum tricornata*; lane 3 - *Saprolegnia ferax*; lane 4 - *Thraustotheca clavata*; lane *5 - Achyla radiosa;* lane 6 - *Saprolegnia turfosa*; lane 7 - *Brevilegnia gracilis*; lane 8 - *Pythium sylvaticum**; lane 9 - *Phytophthora infestans**; lane 10 - *Peronospora viciae**; lanes 11 and 12 - *Peronospora sparsa**; lane 13 *- Albugo candida**; lane 14 - control, no DNA; (* - species belonging to the *Peronosporales*);
Figure 5 shows amplification of DNA from 18 species of *Phytophthora* and *Pythium* by the *Peronosporales* primer DC6 and ITS4: lane 1 - markers; lane 2 - control, no DNA; lane 3 *Phytophthora fragariae*; lane 4 - *Phytophthora capsici*; lane 5 - *Phytophthora palmivora*; lane 6 - *Phytophthora drechsleri*; lane 7 - *Phytophthora megasperma*; lane 8 - *Phytophthora cryptogea*; lane 9 - *Phytophthora cinnamomi*; lane 10 - *Phytophthora syringae*; lane 11 - *Phytophthora citricola*; lane 12 - *Phytophthora cambivora*; lane 13- *Phytophthora nicotianae*; lane 14 - *Phytophthora idaei*; lane 15 - *Phytophthora erythroseptica*; lane 16 - *Phytophthora citrophthora*; lane 17 - *Phytophthora infestans*; lane 18 *-Phytophthora cactorum*; lane 19 - *Pythium sylvaticum*; lane 20 - *Pythium ultimum*;

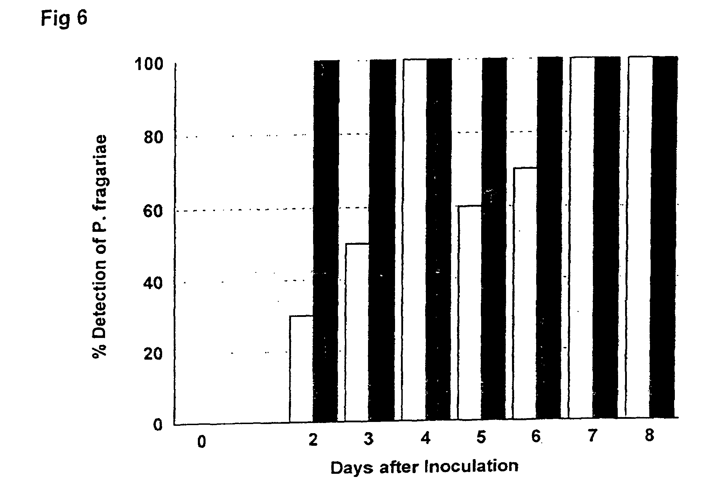

Figure 6 shows a graph indicating detection of infection of strawberry roots by *Phytophthora fragariae* var. *fragariae* by direct PCR (white) with specific primers for *P. fragariae*, and Nested PCR (black) first with the *Peronosporales* primer and ITS4 primers, then the same specific primers; and

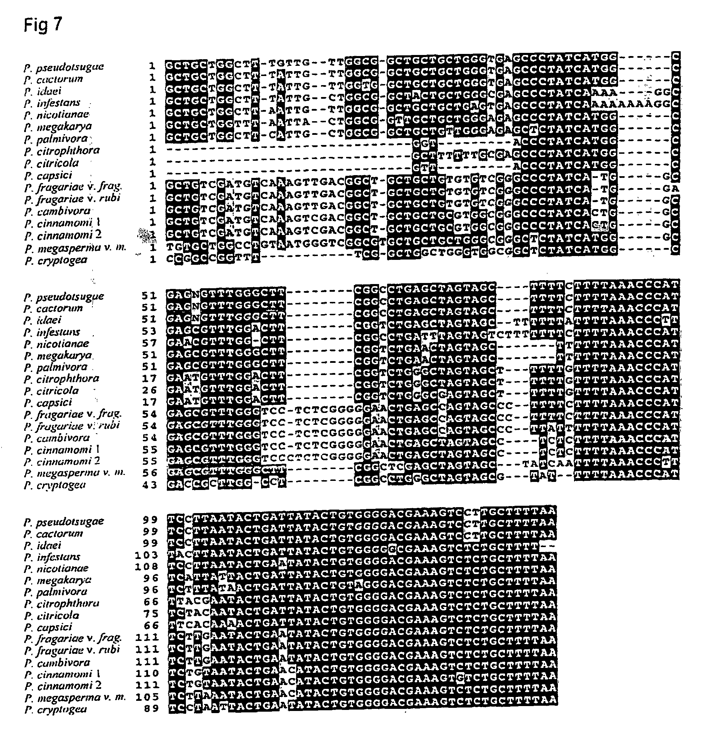

Figure 7 shows aligned DNA sequences of the ITS1 regions of various *Phytophthora* species.

### Materials and methods:

1. Culture of Fungi: Fungi were maintained on oatmeal agar sloped cultures at 4°C. Mycelium, grown on French Bean agar medium at 20°C in the dark, was peeled off and used to inoculate 20ml of a defined sucrose/asparagine/mineral salts medium, at 20°C in the dark. After lwk, the mycelium was harvested by filtration through filter paper, washed with distilled water and stored (-20°C). Zoospores were produced by placing agar plugs of mycelium in a water extract of soil-less compost for 3d at 14°C in the dark with the extract being changed every day. On the last day the extract was replaced with distilled and after incubation overnight at 4°C and four hours at room temperature, the zoospores were collected.
2. Plants: Strawberry plants, cv Alexandria, were grown from seed in pots for 6 weeks. Immediately before inoculation, water was poured into the pot to soak the soil. The plants were then inoculated with a suspension of 25,000 zoospores in 50ml of water and at various time intervals; thereafter plants were removed from pots, their roots were washed in the tap water and two cm long root fragments were cut and examined under a fluorescence microscope or used directly for DNA extraction.
3. DNA extraction: Fungal DNA was extracted using a Nucleon II extraction kit (Scotlab Ltd, Glasgow, UK) according to the manufacturer's recommendations. Between 150 and 250mg of compressed wet-weight mycelium were extracted in a protocol scaled down for use in 1.5ml Eppendorf tubes; this yielded 30 to 50 µg of DNA. Plant DNA was extracted from two or three 2 cm long root fragments.
4. PCR Conditions: Reaction mixtures varied according to the primers, as did the cycling times and temperatures. Generally primers were used at 1mM with 0.2mM of the four dNTPs, 1mg ml-1, 0.05 units of *Taq* polymerase per ul of reaction and the PCR buffer supplied with the enzyme (20mM Tris HCI pH 8.4, 50 mM KCI, 1.5 mM MgCl₂). Test DNA (0.5µl) was added at the end in the reaction mix. In the case of the nested PCR, the amplification product obtained after the first round PCR was diluted one hundred times in sterile water and 0.5 µl was added to the second round PCR. Depending on requirements, the final volume of PCR reaction could be 25, 50 or 100 µl.

The thermocycler parameters for direct and nested PCR were as follows. DNA was denatured at 94°C for 3 min. For direct PCR with primers 1 and 2, the tubes were subjected to 25 cycles of denaturation 94°C, 30s., annealing 65°C, 15s and synthesis, 72°C. For nested PCR in the first round with primers primer 8 and ITS4, DNA was denatured at 94°C for 3 min, followed by 30 cycles of denaturation 94°C, 30s., annealing 55°C, 15s. and synthesis, 72°C for 1 min. 30s. The reaction was finished by leaving the tubes for 10 min. at 72°C. In the second round with primers 1 and 2, DNA was denatured at 94°C for 3 min. followed by 23 cycles of denaturation 94°C, 30s., annealing 65°C, 15s. and synthesis, 72°C 1 min, 30s. The reaction was finished by leaving the tubes for 10 min. at 72°C.

The results of the PCR reaction were assayed by electrophoresis of the PCR products on a TAE 1X (40mM Tris-acetate, 1mM EDTA) agarose gel, staining with ethidium bromide (0.5 µg/ml) and illuminating with UV light.

### Examples

Ribosomal ITS regions and flanking sequences shown in SEQ:ID:Nos 12-39 were sequenced and oligonucleotides were synthesised by standard chemical methods to allow specific amplification of ITS (or flanking) regions of chosen *Phytophthora* and other fungal spp. Such primers allow a single step identification and detection system in which the presence of a PCR-amplified DNA product confirms the presence and identity of the pathogen. Details of the primers are shown in table 1 below:

**Table 1**

| **ORDER/SPECIES** | **PRIMERS/SEQ:ID:No** | | | |
|---|---|---|---|---|
| | Forward | SEQ:No | Reverse | SEQ:No |
| *P.cactorum* | ADF1 | 6 | ADR1 | 7 |
| *P.cambivora* | DC4 | 3 | DC5 | 2 |
| *P.cinnamomi* | DC9 | 8 | DC5 | 2 |
| *P.cryptogea* | CRYF | 9 | CRYR | 10 |
| *P.fragariae* | DC1 | 1 | DC5 | 2 |
| *P.nicotianae* | DC3 | 4 | DC8 | 5 |
| *Peronosporales* | DC6 | 11 | ITS4 | - |

### Example 1:

Forward primer DC1 corresponding to a unique sequence in the ITS1 region of *Phytophthora fragariae* var *fragariae* rDNA was synthesised and has the sequence shown in SEQ:ID:No 1. Reverse primer DC5 has a sequence shown in SEQ:ID:No 2, and was derived from the ITS2 region of *Phytophthora fragariae* var *fragariae* rDNA. When used together at an appropriate annealing temperature (e.g. 62°C) these primers amplified a band (550bp) from ten isolates of *P.fragariae* var, *fragariae* and four of *P.fragariae* var. *rubi* of different physiological races and/or geographical origins. In contrast, it did not amplify DNA from 16 other species: *P.cactorum*, *P.cambivora*, *P.capsici*, *P.cinnamomi*, *P.citricola*, *P.citrophthora*, *P.cryptogea*, *P.drechsleri, P.erythoseptica*, *P.gonapodyides*, *P.idaei*, *P.ilicis, P.megasperma*, *P.nicotianae*, *P.palmivora* and *P.syringae*. Initially, there was a faint band with *P.syringae* but a slight modification of the conditions eliminated this artefact.

The primers were designed around as few as three base pair differences at their 3' ends, sufficient for specific DNA amplification. DC1 and DC5 were used either in direct PCR for P. *fragariae* or in the second round of nested PCR after primers DC6 and ITS4.

### Example 2:

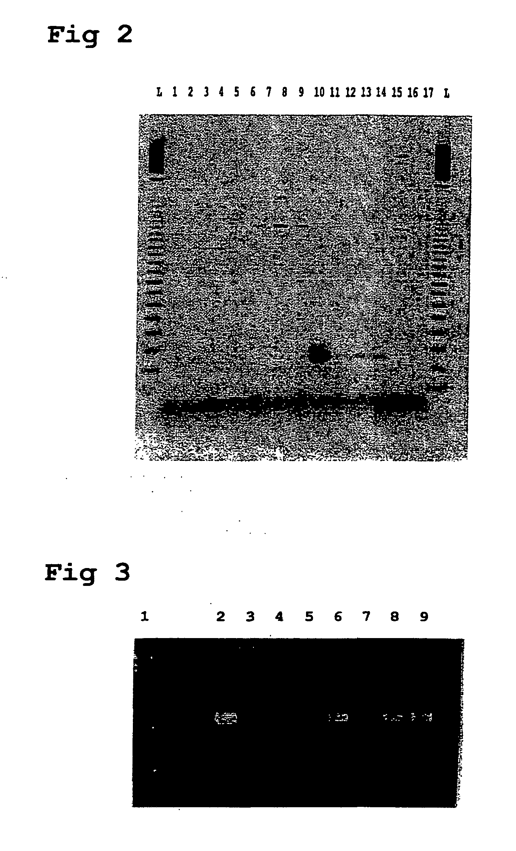

Forward primer DC4 has the sequence shown in SEQ:ID:No 3 and is complimentary to a unique sequence in *Phytophthora cambivora* ITS1 rDNA. When used with reverse primer *ITS2* (see Reference 5) or DC5 above at an appropriate annealing temperature (e.g. 60-62°C) these primers amplify a 530 bp band from DNA obtained from the above species only. DNA from other species showed no amplification products (see fig 2).

### Example 3:

Primer DC3 is derived from a sequence unique to the ITS1 region of *Phytophthora nicotianae* and *P. infestans* (specificity not down to a single species), and has the sequence shown in SEQ:ID:No 4. When used in conjunction with primer *ITS2* or *ITS4* (see Reference 5) at an appropriate annealing temperature (e.g. 58°C) these primers are specific for the above species. DC3 also amplifies a band specifically from *P. nicotianae* DNA when used in conjunction with DC8.

### Example 4:

Primer DC8 (reverse) is derived from the ITS2 region of *P. nicotianae* and has the sequence shown in SEQ:ID:No 5. When used in conjunction with DC3, the primers amplify a band from that species only.

### Example 5:

Primer ADF1 (forward) is derived from ITS1 region of *P. cactorum* and had the sequence shown in SEQ:ID:No 6. Primer ADR1 (reverse) has a sequence shown in SEQ:ID:No 7 and is derived from a sequence of ITS2 rDNA from *Phytophthora cactorum.* Used in conjunction, these primers amplify a band from *P. cactorum* DNA. Primers ADF1 and ADR1 do not distinguish between *P. cactorum* and the very closely related *P.idaei* which occurs commonly on raspberry. However, there is a single base pair sequence difference between the products from these two species that generates a unique restriction site with *Bst* EII, a DNA restriction enzyme: the product of *P.idaei* is cut into two pieces whereas the product from *P.cactorum* is unaffected, and the two PCR products can thereby be distinguished. Primers ADF1 and ADF2 do not amplify DNA from any of the other species listed above.

When used with a primer in ITS1 (8), ADR1 is specific for group 1 species of *Phytopthora* (ie *P. cactorum*, *P.idaei* and *P. pseudotsugae*).

### Example 6:

Primer DC9 is a forward primer derived from the ITS 1 region of *P. cinammomi,* and has the sequence shown in SEQ:ID:No 8. DC9 amplifies a band of DNA from *P. cinammomi* specifically, when used in conjunction with DC5.

### Example 7:

Primer CRYF (forward) has a sequence shown in SEQ:ID:No 9 and is derived from a unique site in the ITS1 rDNA of *P*. *cryptogea*. Primer CRYR is a reverse primer from the ITS2 region of the same organism having the sequence shown in SEQ:ID:No 10. Used together, these two primers have detected *P. cryptogea* in tomato stem bases infected with *P. cryptogea* and in the rock wool in which the plants had been growing (see Fig 3). In addition, they have not amplified DNA from any other *Phytophthora* spp. mentioned above.

### Example 8:

Primer DC6, having the sequence shown in SEQ:ID:No 11, was obtained by comparing sequences of the 18S gene of rDNA of members of the *Oomycota* including fungi belonging to the *Peronosporales*, other orders, plants and other fungi. A forward primer was then designed that would react only with members of the *Peronosporales* when used in conjunction with the published universal primer ITS4, 5'-CAGGGACTTTGGGTAATCA (White et al., 1990), whose sequence is derived from the beginning of the 28S rDNA gene, and which amplifies a 1500bp product containing both ITS1 and ITS2 rDNA sequences of all tested fungi belonging to the order *Peronosporales* (*Pythium ultimum* and *Pythium sylvaticum*, and tissue from a range of plant species infected with the downy mildews, *Peronospora viciae*, *Peronospora sparsa*, *Peronospora parasitica* or *Albugo candida*), except *Bremia lactucae*, a downy mildew. No amplification was obtained with isolates of *Achlya* spp, *Saprolegnia* spp and *Thraustotheca* spp., which belong to other orders of the Oomycetes or with healthy plants.

DC6 amplified a product with a number of other downy mildews including several *Peronospora* spp. and *Albugo candida.* DC6 does not amplify a product with other fungi, even those belonging to the Oomycota, if they are not members of the order Peronosporales (Figure 4 A, B). Downy mildews are obligate pathogens and always found in close association with their hosts, nevertheless only one PCR product was obtained when DNA from plants infected with downy mildews was amplified, and that product came from the downy mildews not the plants. With universal primers, two products were always obtained, one from the plant and one from the fungus.

The universality of application of this primer to *Phytophthora* and *Pythium* species can be seen in Figure 5. The partial failure with *P. palmivora* observed in lane 5 did not occur in later experiments.

Figure 6 shows clearly the improvement in detection in infected plant material (in this case strawberry roots) of Nested PCR using the *Peronosporales* DC6 primer over Direct PCR with the specific primer alone; no [Mplification was obtained by either method with DNA from roots from the controls. By two days, only 30% of the samples from inoculated plants gave a product with direct PCR but by four days this had risen to 100% and although it declined on days 5 and 6 (60-70%), it rose again to 100% by the end of the experiment. In contrast all samples from 2 days onwards were positive with nested PCR.

Nested PCR was also used with DNA samples extracted two hours, 16 hours and one day after inoculation. *P.fragariae* was detected in all samples taken one day after inoculation but only in some roots after 16 hours and in none after two hours.

Fig 7 shows a listing of DNA sequences of the ITS1 regions of various *Phytophthora* species, aligned to show the regions of conservation (in black) and the regions of variation (in white). When constructing primers to distinguish one or a group of species from the remaining species, a skilled man can choose an oligonucleotide sequence which varies or is constant throughout the species of interest. For example, an oligonucleotide including the sequence 5'-TCTCGGG will distinguish the group of *P*. *fragariae*, *P.cambivora* and *P.cinammomi* from the others. Using nested PCR techniques, or an appropriate reverse primer specific for one of these selected species, the selected group can be reduced to a single species.

Modifications and improvements may be incorporated without departing from the scope of the invention.

### References

The following documents are incorporated herein by reference:
1 Goodwin P. H., Kirkpatrick B. C. and Duniway J. M. (1989). *Phytopathology* 79, 716-721.
2 Lee S. B. and Taylor J. W. (1992) *Journal of Molecular Biology and Evolution* 9, 636-653.
3 White T. J.et al (1990) In *PCR Protocols* (ed. M. A. Innis, D. H. Gelfand, J. J. Sninsky and T. J. White) 315-322, Academic Press: San Diego.
4 Berbee, M. L. & Taylor, J. W. (1992). *Canadian Journal of Botany* 71, 1114-1127.
5 Gray, M. W., et al (1984). *Nucleic Acids Research* 12, 5834-5852
6 Nues, R. W. et al (1994). *Nucleic Acids Research* 22, 912-919.
7 Sherriff C., et al (1994). *Experimental Mycology* 18, 121-138.
8 Zambino, P. J. & Szabo, L. J. (1993). *Mycologia* 85, 401-414.
9 Gunderson, J. H. et al (1987). *PNAS USA.* 84, 5823-5827.
10 British Patent Application No 9509112.0.

## Claims

1. A polynucleotide having a sequence corresponding to any one of SEQ:ID:Nos 1 to 39 or a complementary strand thereof.

2. The use of a polynucleotide according to Claim 1, for the identification of fungal nucleic acid by the binding between the polynucleotide and a target nucleic acid sequence of the fungus.

3. The use of a polynucleotide according to Claim 2 in a nucleotide hybridisation assay.

4. The use of a polynucleotide according to Claim 3 in a PCR assay.

5. The use of a polynucleotide according to any one of Claims 2 to 4, in which combinations of more than one polynucleotide according to Claim 1 are used in the assay.

6. The use of a polynucleotide according to Claim 5, in which only one of the polynucleotides complements a unique sequence in the target nucleic acid to be identified.

7. The use of a polynucleotide according to Claim 5, in which each polynucleotide is complementary to a unique sequence in the target nucleic acid to be identified.

8. The use of a polynucleotide according to any one of Claims 2 to 7, wherein the target nucleic acid sequence is duplicated.

9. The use of a polynucleotide according to any one of Claims 4 to 8, wherein a first combination of polynucleotides is used to amplify a first sequence of fungal nucleic acid, and a second combination of polynucleotides is used to amplify from said first sequence a second sequence of fungal nucleic acid contained within said first sequence.

10. The use of a polynucleotide according to Claim 9, wherein the first combination of polynucleotides is selected to amplify a fungal nucleic acid sequence common to several species of fungus, and the second combination of polynucleotides is selected to amplify a fungal nucleic acid sequence specific to one species of fungus.

11. A method of detecting the presence of nucleic acid characteristic of a species of fungus, the method comprising providing a sample of material to be tested which is suspected of containing or consisting of nucleic acid from said species of fungus, providing a polynucleotide according to Claim 1 having a sequence complementary to at least a part of the species-specific sequence of ITS region DNA or ITS flanking region DNA of said species of fungus, exposing the polynucleotide to the material, and determining whether reaction between the polynucleotide and the sample of material has occurred.

12. A method of detecting the presence of a fungus and/or determining the fungal species responsible for fungal infection in organic matter, the method comprising providing a sample of material to be tested which is suspected of containing or consisting of nucleic acid from said species of fungus, providing a polynucleotide according to Claim 1 having a sequence complementary to at least a part of the species-specific sequence of ITS region DNA or ITS flanking region DNA of said species of fungus, exposing the polynucleotide to the material, determining whether reaction between the polynucleotide and the sample of material has occurred, and determining the species of fungus present from the results of said reaction.

13. A method according to Claim 11 or 12, wherein the reaction comprises binding of the polynucleotide and the material and amplification of nucleic acid.

14. Use as claimed in any one of Claims 2 to 10, or a method as claimed in any one of Claims 11 to 13, wherein the fungus in a member of the order *Peronosporales*.

## Patentansprüche

1. Ein Polynukleotid mit einer Sequenz, die einer der SEQ: ID: Nr. 1 bis 39 oder einem komplementären Strang davon entspricht.

2. Die Verwendung eines Polynukleotids gemäß Anspruch 1 zur Identifizierung von Pilz-Nukleinsäure durch das Binden zwischen dem Polynukleotid und einer Ziel-Nukleinsäuresequenz des Pilzes.

3. Verwendung eines Polynukleotids gemäß Anspruch 2 in einem Nukleotid-Hybridisierungsassay.

4. Verwendung eines Polynukleotids gemäß Anspruch 3 in einem PCR-Assay.

5. Verwendung eines Polynukleotids gemäß einem der Ansprüche 2 bis 4, wobei in dem Assay Kombinationen aus mehr als einem Polynukleotid gemäß Anspruch 1 verwendet werden.

6. Verwendung eines Polynukleotids gemäß Anspruch 5, wobei nur eines der Polynukleotide eine einzigartige Sequenz in der zu identifizierenden Ziel-Nukleinsäure ergänzt.

7. Verwendung eines Polynukleotids gemäß Anspruch 5, wobei jedes Polynukleotid zu einer einzigartigen Sequenz in der zu identifizierenden Ziel-Nukleinsäure komplementär ist.

8. Verwendung eines Polynukleotids gemäß einem der Ansprüche 2 bis 7, wobei die Ziel-Nukleinsäuresequenz dupliziert wird.

9. Verwendung eines Polynukleotids gemäß einem der Ansprüche 4 bis 8, wobei eine erste Kombination aus Polynukleotiden verwendet wird, um eine erste Sequenz Pilz-Nukleinsäure zu amplifizieren, und wobei eine zweite Kombination aus Polynukleotiden verwendet wird, um aus dieser ersten Sequenz eine zweite Sequenz Pilz-Nukleinsäure, die in der ersten Sequenz enthalten ist, zu amplifizieren.

10. Verwendung eines Polynukleotids gemäß Anspruch 9, wobei die erste Kombination aus Polynukleotiden ausgewählt wird, um eine Pilz-Nukleinsäuresequenz, die verschiedenen Pilzarten gemein ist, zu amplifizieren, und wobei die zweite Kombination aus Polynukleotiden ausgewählt wird, um eine Pilz-Nukleinsäuresequenz, die für eine Pilzart spezifisch ist, zu amplifizieren.

11. Ein Verfahren zur Wahrnehmung der Anwesenheit der Nukleinsäurecharakteristik einer Pilzart, wobei das Verfahren das Bereitstellen einer Probe eines zu untersuchenden Materials, von dem vermutet wird, dass es Nukleinsäure aus der Pilzart enthält oder aus dieser besteht, das Bereitstellen eines Polynukleotids gemäß Anspruch 1 mit einer Sequenz, die zu mindestens einem Teil der artspezifischen Sequenz der ITS-Region DNA oder ITS flankierenden Region DNA der Pilzart komplementär ist, das In-Kontakt-Bringen des Polynukleotids mit dem Material und das Bestimmen, ob zwischen dem Polynukleotid und der Probe des Materials eine Reaktion statt gefunden hat, beinhaltet.

12. Verfahren zur Wahrnehmung der Anwesenheit eines Pilzes und/oder zur Bestimmung der Pilzart, die für die Pilzinfektion in organischer Substanz verantwortlich ist, wobei das Verfahren das Bereitstellen einer Probe eines zu untersuchenden Materials, von dem vermutet wird, dass es Nukleinsäure aus der Pilzart enthält oder aus dieser besteht, das Bereitstellen eines Polynukleotids gemäß Anspruch 1 mit einer Sequenz, die zu mindestens einem Teil der artspezifischen Sequenz der ITS-Region DNA oder ITS flankierenden Region DNA der Pilzart komplementär ist, das In-Kontakt-Bringen des Polynukleotids mit dem Material, das Bestimmen, ob zwischen dem Polynukleotid und der Probe des Materials eine Reaktion statt gefunden hat, und das Bestimmen der Pilzart, die aus den Ergebnissen dieser Reaktion hervorgeht, beinhaltet.

13. Verfahren gemäß Anspruch 11 oder 12, wobei die Reaktion das Binden des Polynukleotids mit dem Material und die Amplifikation von Nukleinsäure beinhaltet.

14. Verwendung gemäß einem der Ansprüche 2 bis 10 oder Verfahren gemäß einem der Ansprüche 11 bis 13, wobei der Pilz der Ordnung *Peronosporales* angehört.

## Revendications

1. Un polynucléotide ayant une séquence correspondant à n'importe laquelle des SEQ : ID : Nos 1 à 39 ou un brin complémentaire de celui-ci.

2. L'utilisation d'un polynucléotide selon la revendication 1, pour identifier un acide nucléique fongique grâce à la liaison entre le polynucléotide et une séquence d'acide nucléique cible du champignon.

3. L'utilisation d'un polynucléotide selon la revendication 2 dans un test d'hybridation de nucléotides.

4. L'utilisation d'un polynucléotide selon la revendication 3 dans un test de réaction en chaîne de la polymérase.

5. L'utilisation d'un polynucléotide selon n'importe laquelle des revendications 2 à 4, dans laquelle des combinaisons de plus d'un polynucléotide selon la revendication 1 sont utilisées dans le test.

6. L'utilisation d'un polynucléotide selon la revendication 5, dans laquelle seul l'un des polynucléotides complète une séquence unique dans l'acide nucléique cible à identifier.

7. L'utilisation d'un polynucléotide selon la revendication 5, dans laquelle chaque polynucléotide est complémentaire à une séquence unique dans l'acide nucléique cible à identifier.

8. L'utilisation d'un polynucléotide selon n'importe laquelle des revendications 2 à 7, dans laquelle la séquence d'acide nucléique cible est dupliquée.

9. L'utilisation d'un polynucléotide selon n'importe laquelle des revendications 4 à 8, dans laquelle une première combinaison de polynucléotides sert à amplifier une première séquence d'acide nucléique fongique, et une deuxième combinaison de polynucléotides sert à amplifier à partir de ladite première séquence une deuxième séquence d'acide nucléique fongique contenue dans ladite première séquence.

10. L'utilisation d'un polynucléotide selon la revendication 9, dans laquelle la première combinaison de polynucléotides est sélectionnée afin d'amplifier une séquence d'acide nucléique fongique commune à plusieurs espèces de champignon, et la deuxième combinaison de polynucléotides est sélectionnée afin d'amplifier une séquence d'acide nucléique fongique spécifique à une espèce de champignon.

11. Un procédé de détection de la présence d'acide nucléique caractéristique d'une espèce de champignon, le procédé comportant le fait de fournir un échantillon de matériau à tester que l'on soupçonne de contenir ou de consister en de l'acide nucléique provenant de ladite espèce de champignon, le fait de fournir un polynucléotide selon la revendication 1 ayant une séquence complémentaire à au moins une partie de la séquence spécifique à l'espèce de l'ADN dans la région ITS ou de l'ADN dans la région flanquante de l'ITS de ladite espèce de champignon, le fait d'exposer le polynucléotide au matériau, et le fait de déterminer si la réaction entre le polynucléotide et l'échantillon de matériau s'est produite.

12. Un procédé de détection de la présence d'un champignon et/ou de détermination de l'espèce fongique responsable de l'infection fongique dans de la matière organique, le procédé comportant le fait de fournir un échantillon de matériau à tester que l'on soupçonne de contenir ou de consister en de l'acide nucléique provenant de ladite espèce de champignon, le fait de fournir un polynucléotide selon la revendication 1 ayant une séquence complémentaire à au moins une partie de la séquence spécifique à l'espèce de l'ADN dans la région de l'ITS ou de l'ADN dans la région flanquante de l'ITS de ladite espèce de champignon, le fait d'exposer le polynucléotide au matériau, le fait de déterminer si la réaction entre le polynucléotide et l'échantillon de matériau s'est produite, et le fait de déterminer l'espèce de champignon présente d'après les résultats de ladite réaction.

13. Un procédé selon la revendication 11 ou la revendication 12, dans lequel la réaction comporte la liaison du polynucléotide et du matériau et l'amplification de l'acide nucléique.

14. Utilisation telle que revendiquée dans n'importe laquelle des revendications 2 à 10, ou un procédé tel que revendiqué dans n'importe laquelle des revendications 11 à 13, dans lequel le champignon fait partie de l'ordre des *Peronosporales*.


### SEQUENCE LISTING

(1) GENERAL INFORMATION:
   (i) APPLICANT:
      (A) NAME: Scottish Crop Research Institute
      (B) STREET: Invergowrie
      (C) CITY: Dundee
      (E) COUNTRY: United Kingdom
      (F) POSTAL CODE (ZIP): DD2 5DA
      (G) TELEPHONE: 01382 562731
      (H) TELEFAX: 01382 562426
   (ii) TITLE OF INVENTION: Detection and identification of fungi
   (iii) NUMBER OF SEQUENCES: 39
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
   (vi) PRIOR APPLICATION DATA:
      (A) APPLICATION NUMBER: GB 9509112.0
      (B) FILING DATE: 04-MAY-1995
(2) INFORMATION FOR SEQ ID NO: 1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora fragariae var fragariae
   (viii) POSITION IN GENOME:
      (B) MAP POSITION: Based on ITS1 region
   (xi) SEQUENCE DESCRIPTION: SEQ ID NO: 1:
   ACTTAGTTGG GGGCCTGTCT 20
(2) INFORMATION FOR SEQ ID NO: 2:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora fragariae var fragariae
   (viii) POSITION IN GENOME:
      (B) MAP POSITION: based on ITS2 region
   (xi) SEQUENCE DESCRIPTION: SEQ ID NO: 2:
   CGCCGACTGG CCACACAG 18
(2) INFORMATION FOR SEQ ID NO: 3:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cambivora
   (viii) POSITION IN GENOME:
      (B) MAP POSITION: based on ITS1 region
   (xi) SEQUENCE DESCRIPTION: SEQ ID NO: 3:
   TTAGTTGGGG GCTAGTCCC 19
(2) INFORMATION FOR SEQ ID NO: 4:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora nicotianae and Phytophthora infestans
   (viii) POSITION IN GENOME:
      (B) MAP POSITION: based on ITS1 region
   (xi) SEQUENCE DESCRIPTION: SEQ ID NO: 4:
   CCAATAGTTG GGGGTCTTAT T 21
(2) INFORMATION FOR SEQ ID NO: 5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: P. nicotianae
   (viii) POSITION IN GENOME:
      (B) MAP POSITION: Derived from ITS2 region
   (xi) SEQUENCE DESCRIPTION: SEQ ID NO: 5:
   AATTCAAAAG CCAAGCCACC 20
(2) INFORMATION FOR SEQ ID NO: 6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: P. cactorum
   (viii) POSITION IN GENOME:
      (B) MAP POSITION: Derived from ITS1 rDNA
   (xi) SEQUENCE DESCRIPTION: SEQ ID NO: 6:
   TACTGTGGGG ACGAAAGTCC T 21
(2) INFORMATION FOR SEQ ID NO: 7:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cactorum
   (viii) POSITION IN GENOME:
      (B) MAP POSITION: based on ITS2 region
   (xi) SEQUENCE DESCRIPTION: SEQ ID NO: 7:
   CCGATTCAAA AGCCAAGCAA CT 22
(2) INFORMATION FOR SEQ ID NO: 8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cinnamomi
   (viii) POSITION IN GENOME:
      (B) MAP POSITION: Derived from ITS1 rDNA
   (xi) SEQUENCE DESCRIPTION: SEQ ID NO: 8:
   AACTGAGCTA GTAGCCTCTC 20
(2) INFORMATION FOR SEQ ID NO: 9:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cryptogea
   (viii) POSITION IN GENOME:
      (B) MAP POSITION: derived from ITS1 rDNA
   (xi) SEQUENCE DESCRIPTION: SEQ ID NO: 9:
   CGGTTTTCGG CTGGCTGGG 19
(2) INFORMATION FOR SEQ ID NO: 10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cryptogea
   (viii) POSITION IN GENOME:
      (B) MAP POSITION: derived from ITS2 rDNA
   (xi) SEQUENCE DESCRIPTION: SEQ ID NO: 10:
   CAGCTTGCGC CAGAACAGAC 20
(2) INFORMATION FOR SEQ ID NO: 11:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Perinosporales spp.
   (xi) SEQUENCE DESCRIPTION: SEQ ID NO: 11:
   GAGGGACTTT TGGGTAATCA 20
(2) INFORMATION FOR SEQ ID NO: 12:
   (i) SEQUENCE CHARACTERISTICS:
      (A) LENGTH: 220 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora infestans
      (C) INDIVIDUAL ISOLATE: 89/AF1
   (viii) POSITION IN GENOME:
      (B) MAP POSITION: ITS1
   (xi) SEQUENCE DESCRIPTION: SEQ ID NO: 12:
   GGATCATTAC CACACCTAAA AACTTTCCAC GTGAACCGTT TCAACCCAAT AGTTGGGGGT 60
   CTTACTTGGC GGCGGCTGCT GGCTTTATTG CTGGCGGCTA CTGCTGGGCG AGCCCTATCA 120
   AAAGGCGAGC GTTTGGACTT CGGTCTGAGC TAGTAGCTTT TTTATTTTAA ACCCTTTACT 180
   TAATACTGAT TATACTGTGG GGGCGAAAGT CTCTGCTTTT 220
(2) INFORMATION FOR SEQ ID NO: 13:
   (i) SEQUENCE CHARACTERISTICS:
      (A) LENGTH: 240 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora fragariae var fragariae
      (C) INDIVIDUAL ISOLATE: 168
   (viii) POSITION IN GENOME:
      (B) MAP POSITION: ITS1
   (xi) SEQUENCE DESCRIPTION: SEQ ID NO: 13:
   CCACACCTAA AAACTTTCCA CGTGAACCGT ATCAACCCAC TTAGTTGGGG GCCTGTCTGG 60
   CGGCTGGCTG TCGATGTCAA AGTTGACGGC TGCTGCTGTG TGTCGGGCCC TATCATGGCG 120
   AGCGTTTGGG TCCTCTCGGG GGAACTGAGC CAGTAGCCCT TTTCTTTTAA ACCCATTCTT 180
   GAATACTGAA TATACTGTGG GGACGAAAGT CTCTGCTTTT AACTAGATAG CAACTTTCAG 240
(2) INFORMATION FOR SEQ ID NO: 14:
   (i) SEQUENCE CHARACTERISTICS:
      (A) LENGTH: 222 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora megasperma
      (C) INDIVIDUAL ISOLATE: MEG1
   (viii) POSITION IN GENOME:
      (B) MAP POSITION: ITS1
   (xi) SEQUENCE DESCRIPTION: SEQ ID NO: 14:
   AACTTCCACG TGACCGTATC AACCCCTAAT TGGGGCTTGC TCGGCGGCGT GTGTGCTGGC 60
   CTGTAATGGG TCGGCGTGCT GCTGCTGGGC GGGCTCTATC ATGGGGCGAN GTTTGGGCTT 120
   CGGCTCGAGC TAGTAGCTAT CAATTTTAAA CCCTTTCTTA AATACTTGAA CATACTGTGG 180
   GGACGAAAGT CTCTGCTAAA AGGCTAGATA GCAACTTTCA GC 222
(2) INFORMATION FOR SEQ ID NO: 15:
   (i) SEQUENCE CHARACTERISTICS:
      (A) LENGTH: 235 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cambivora
      (C) INDIVIDUAL ISOLATE: CAM1
   (viii) POSITION IN GENOME:
      (B) MAP POSITION: ITS1
   (xi) SEQUENCE DESCRIPTION: SEQ ID NO: 15:
   AAAAAACTTT CCACGTGAAC CGTATCAACC CACTTAGTTG GGGGCTAGTC CCGGCGGCTG    60
   GCTGTCGATG TCAAAGTTGA CGGCTGCTGC TGTGTGTCGG GCCCTATCAT GGCGAGCGTT   120
   TGGGTCCTCT CGGGGGAACT GAGCCAGTAG CCCTTATTTT TTAAACCCAT TCTTGAATAC   180
   TGAATATACT GTGGGGACGA AAGTCTCTGC TTTTAACTAG ATAGCAACTT TCAGC        235
(2) INFORMATION FOR SEQ ID NO: 16:
   (i) SEQUENCE CHARACTERISTICS:
      (A) LENGTH: 229 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cryptogea
      (C) INDIVIDUAL ISOLATE: CRY3
   (viii) POSITION IN GENOME:
      (B) MAP POSITION: ITS1
   (xi) SEQUENCE DESCRIPTION: SEQ ID NO: 16:
   AAGGATCATT ACCACACCTA AAAAACTTTC CACGTGAACC GTATCAACCT TTTTAATTTG    60
   GGGGCTTCCG TCTGGCCGGC CGGTTTTCGG CTGGCTGGGT GGCGGCTCTA TCATGGCGAC   120
   CGCTTGGCCT CGGCCTGGGC TAGTAGCGTA TTTTTAAACC CATTCCTAAT TACTGAATAT   180
   ACTGTGGGGA CGAAAGTCTC TGCTTTTAAC TAGATAGCAA CTTTCAGCA               229
(2) INFORMATION FOR SEQ ID NO: 17:
   (i) SEQUENCE CHARACTERISTICS:
      (A) LENGTH: 189 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora citricola
      (C) INDIVIDUAL ISOLATE: CIT2
   (viii) POSITION IN GENOME:
      (B) MAP POSITION: ITS1
   (xi) SEQUENCE DESCRIPTION: SEQ ID NO: 17:
   CTAAAAAACT TTTCACGTGA ACCGTATCAA CCCTTTTAGT TGGGGGTCTT GCTTTTTTGC    60
   GAGCCCTATC ATGGCGAATG TTTGGACTTC GGTCTGGGCT AGTAGCTTTT TGTTTTAAAC   120
   CCATTCTACA ATACTGATTA TACTGTGGGG ACGAAAGTCT CTGCTTTTAA CTAGATAGCA   180
   ACTTTCAGC                                                           189
(2) INFORMATION FOR SEQ ID NO: 18:
   (i) SEQUENCE CHARACTERISTICS:
      (A) LENGTH: 230 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora nicotianae
      (C) INDIVIDUAL ISOLATE: NIC1
   (viii) POSITION IN GENOME:
      (B) MAP POSITION: ITS1
   (xi) SEQUENCE DESCRIPTION: SEQ ID NO: 18:
   AAAAACTTTC CACGTGAACC GTTTCAACCC AATAGTTGGG GGTCTTATTT GGCGGCGGCT    60
   GCTGGCTTAA TTGTTGGCGG CTGCTGCTGA GTGAGCCCTA TCAAAAAAAA GGCGAACGTT   120
   TGGCTTCGGC CTGATTTAGT AGTCTTTTTT TCTTTTAAAC CCATTCCTTA ATACTGAATA   180
   TACTGTGGGG ACGAAAGTCT CTGCTTTTAA CTAGATAGCA ACTTTCAGCA              230
(2) INFORMATION FOR SEQ ID NO: 19:
   (i) SEQUENCE CHARACTERISTICS:
      (A) LENGTH: 209 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora pseudotsugae
      (C) INDIVIDUAL ISOLATE: PSE1
   (viii) POSITION IN GENOME:
      (B) MAP POSITION: ITS1
   (xi) SEQUENCE DESCRIPTION: SEQ ID NO: 19:
   CACCTAAAAC TTTCCACGTG AACCGTTTCA AACCAAATAG TTNNGGGTCT TGTCTGGTGG 60
   CGGCTGCTGG CTTTGTTGTT GGCGGCTGCT GCTGGGTGAG CCCTATCATG GCGAGNGTTT 120
   GGGCTTCGGC CTGAGCTAGT AGCTTTTCTT TTAAACCCAT TCCTTAATAC TGATTATACT 180
   GTGGGGACGA AAGTCCTTGC TTTTAACTA 209
(2) INFORMATION FOR SEQ ID NO: 20:
   (i) SEQUENCE CHARACTERISTICS:
      (A) LENGTH: 194 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora idaei
      (C) INDIVIDUAL ISOLATE: IDA4
   (viii) POSITION IN GENOME:
      (B) MAP POSITION: ITS1
   (xi) SEQUENCE DESCRIPTION: SEQ ID NO: 20:
   AAAAACTTTC CACGTGAACC GTTTCAAACC AAATAGTTGG GGGTCTTGTC TGGTGGCGGC 60
   TGCTGGCTTT ATTGTTGGTG GCTGCTGCTG GGTGAGCCCT ATCATGGCGA GNGTTTGGGC 120
   TTCGGCCTGA GCTAGTAGCT TTTCTTTTAA ACCCATTCCT TAATACTGAT TATACTGTGG 180
   GGACGAAAGT CCTT 194
(2) INFORMATION FOR SEQ ID NO: 21:
   (i) SEQUENCE CHARACTERISTICS:
      (A) LENGTH: 518 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora infestans
      (C) INDIVIDUAL ISOLATE: 89/AF1
   (viii) POSITION IN GENOME:
      (B) MAP POSITION: ITS2
   (xi) SEQUENCE DESCRIPTION: SEQ ID NO: 21:
   GCAGGATTCA GTGAGTCATC GAAANNTNGA ACGCATATTG CACTTCCGGG TTAGTCCTGG    60
   AAGTATGCCT GTATCAGTGT CCGTACAACA AACTTGGCTT TCTTCCTTCC GTGTAGTCGG   120
   TGGAGGAGAT GCCAGATGTG AAGTGTCTTG CGGTTGGTTT TCGGACCGAC TGCGAGTCCT   180
   TTTAAATGTA CTAAACTGTA CTTCTCTTTG CTCCAAAAGT GGTGGCACTT GCTGGTTGTG   240
   GACGCTGCTA TTGTAGCNAG TTGGCGACCG GTTTGTCTGC TGCGGCGTTA ATGGAGAAAT   300
   GCTCGATTCG TGGTATGGTT GGCTTCGGCT GAACAATGCG CTTATTGGGT GATTTTCCTG   360
   CTGTGGCGTG ATGGACTGGT GAACCATGGC TCTTTAGCTT GGCATTTGAA TCGGCTTTGC   420
   TGTTGCGAAG TAGAGTGGCG GCTTCGGCTG CCGAGGGTCG ATCCATTTGG GAAATGTTGT   480
   GTACTTCGGT ATGCATCTCA ATTGGACCTG ATATCAGG                           518
(2) INFORMATION FOR SEQ ID NO: 22:
   (i) SEQUENCE CHARACTERISTICS:
      (A) LENGTH: 528 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora fragariae var fragariae
      (C) INDIVIDUAL ISOLATE: 168
   (viii) POSITION IN GENOME:
      (B) MAP POSITION: ITS2
   (xi) SEQUENCE DESCRIPTION: SEQ ID NO: 22:
   TGAGTCATCG AAATTTTGAA CGCATATTGC ACTTCCGGGT TAGTCCTGGG AGTATGCCTG    60
   TATCAGTGTC CGTACATCAA ACTTGGCTCT CTTCCTTCCG TGTAGTCGGT GGATGGGGAC   120
   GCCAGACGTG AGGTGTCTTG CGGGTGGCCT TCGGGCTGCC TGCGAGTCCC TTGAAATGTA   180
   CTGAACTGTA CTTCTCTTTG CTCGAAAAGC GTGACGTTGT TGGTTGTGGA GGCTGCCTGT   240
   GTGGCCAGTC GGCGACCGGT TTGTCTGCTG CGGCGTTTAA TGGAGGAGTG TTCGATTCGC   300
   GGTATGGTTG GCTTCGGCTG AACAATGCGC TTATTGGACG TTCTTCCTGC TGTGGCGGTA   360
   CGGATCGGTG AACCGTAGGT GTGCGAGGCT TGGCCTTTGA ACCGGCGGTG TTGGTCGCGA   420
   AGTAGGGTGG CGGCTTCGGC TGTCGAGGGG TCGATCCATT TGGGAACTTG TGTCTCTGCG   480
   GCGCGCTTCG GTGTGCTGCG GGTGGCATCT CAATTGGACC TGATATCG                528
(2) INFORMATION FOR SEQ ID NO: 23:
   (i) SEQUENCE CHARACTERISTICS:
      (A) LENGTH: 524 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora megasperma
      (C) INDIVIDUAL ISOLATE: MEG1
   (viii) POSITION IN GENOME:
      (B) MAP POSITION: ITS2
   (xi) SEQUENCE DESCRIPTION: SEQ ID NO: 23:
   GGATTCAGTG AGTCATCGAA ATTTTGAACG CATATTGCAC TTCCGGGTTA GTCCTGGGAG 60
   TATGCCTGTA TCAGTGTCCG TACATCAACC TTGGTTTTCT TCCTTCCGTG TAGTCGGTGG 120
   AGGATATGCC AGACGTGAAG TGTCTTGCTA GCGGTCTTTC GAGTCTGCCG GTGAGTCCTT 180
   TTAAATGTAC TGAACTGTAC TCTCTCTTTG CGCGAAAAGC GTGGCGTTGC TGGTTGTGGA 240
   GGCTGCCCGT GTGGCCAGTC GGCGACCGGT TTGTTAGCTG TGGCGTTTGG AGGAGTGTTC 300
   GATTCGCGGT ATGGTTGGCT TCGGCTGAAC AATCTGCTTA TTGGGTGCTT TTCCTGTCAT 360
   GGCGGTATGA ACTGGTGAAC CGTAGCTGTN TGGTGCTTGG CTTTTCAACC GGCTTTGCTN 420
   TGCGAAGTAG TGTGGCGGCT TCGGCTGTCG AGGGGTCGAT CCTAAAAGGG AAACTTTGTG 480
   TGTGCGGCTT CGTCGTGCGC GCATCTCAAT TGGACCTGAT ATCA 524
(2) INFORMATION FOR SEQ ID NO: 24:
   (i) SEQUENCE CHARACTERISTICS:
      (A) LENGTH: 521 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cambivora
      (C) INDIVIDUAL ISOLATE: CAM1
   (viii) POSITION IN GENOME:
      (B) MAP POSITION: ITS2
   (xi) SEQUENCE DESCRIPTION: SEQ ID NO: 24:
   GGATTCAGTG AGTCATCGAA ATTTTGAACG CATATTGCAC TTCCGGGTTA GTCCTGGGAG 60
   TATGCCTGTA TCAGTGTCCG TACATCAAAC TTGGCTCTCT TCCTTCCGTG TAGTCGGTGG 120
   ATGGGGACGC CAGACGTGAG GTGTCTTGCG GGTGGNCTTC GGGCTGGCTG CGAGTCCCTT 180
   GAAATGTACT GAACTGTACT TCTCTTTGCT CGAAAAGCGT GACGTTGTTG GTTGTGGAGG 240
   CTGCCTGTGT GGCCAGTCGG CGACCGGTTT GTCTGCTGCG GCGTTTAATG GAGGAGTGTT 300
   CGATTCGCGG TATGGTTGGC TTCGGCTGAA CAATGCGCTT ATTGGACGTT CTTCCTGCTG 360
   TGGCGGTACG GATCGGTGAA CCGTAGCTGT GCGAGGCTTG GCCTTTGAAC CGGCGGTGTT 420
   GGTCGCGAAG TAGGGTGGCG GCTTCGGCTG TCGAGGGGTC GATCCATTTG GGAACTTGTG 480
   TCTCTGCGGC GCGCTTNGGT GTGCTGCGGG TGGCACTCTA A 521
(2) INFORMATION FOR SEQ ID NO: 25:
   (i) SEQUENCE CHARACTERISTICS:
      (A) LENGTH: 513 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cryptogea
      (C) INDIVIDUAL ISOLATE: CRY3
   (viii) POSITION IN GENOME:
      (B) MAP POSITION: ITS2
   (xi) SEQUENCE DESCRIPTION: SEQ ID NO: 25:
   GCATTCAGTG AGTCATCGAA ATTTTGAACG CATATTGCAC TTCCGGGTTA GTCCTGGGAG 60
   TATGCCTGTA TCAGTGTCCG TACACTAAAC TTGGCTCCCT TCCTTCCGTG TAGTCGGTGG 120
   ATGGGGACGC GCAGATGTGA AGTGTCTTGC GGCTGGTCTT CGGTCCGGCT GCGAGTCCTT 180
   TGAAATGTAC TACACTGTAC TTCTCTTTGC TCGAAAAGCG TGACGTTGCT GGTTGTGGAG 240
   GCTGCCTGTG TGGCATGTCG GCGACCGGTT TGTCTGCTGC GGCGTTTAAT GGAGGAGTGT 300
   TCGATTCGCG GTATGGTTGG CTTCGGCTGA ACAGACGCTT ATTGGGTGCT TTTCCTGCTG 360
   TGGCTGGATG GACTGGTGAA CCGTAGCTGT GCTAGGCTTG GCGTTTGAAC CGGCGGTGTG 420
   GTGCGAAGTA GGGTGTCTGT TCTGGCGTAA GCTGGGGTGG ACGAGGGTCG ATCCATTTGG 480
   GAAACGTTGT GNNCTTCGGC GCGCATCTCA ATT 513
(2) INFORMATION FOR SEQ ID NO: 26:
   (i) SEQUENCE CHARACTERISTICS:
      (A) LENGTH: 527 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora drechslerii
      (C) INDIVIDUAL ISOLATE: DRE1
   (viii) POSITION IN GENOME:
      (B) MAP POSITION: ITS2
   (xi) SEQUENCE DESCRIPTION: SEQ ID NO: 26:
   GGATTCAGTG AGTCATCGAA ATTTTGAACG CATATTGCAC TTCCGGGTTA GTCCTGGGAG     60
   TATGCCTGTA TCAGTGTCCG TACACTAAAC TTGGCTCCCT TCCTTCCGTG TAGTCGGTGG    120
   ATGGGGACGC GCAGATGTGA AGTGTCTTGC GGCTGGTCTT CGGTCCGGCT GCGAGTCCTT    180
   TGAAATGTAC TACACTGTAC TTCTCTTTGC TCGAAAAGCG TGACGTTGCT GGTTGTGGAG    240
   GCTGCCTGTG TGGCATGTCG GCGACCGGTT TGTCTGCTGC GGCGTTTAAT GGAGGAGTGT    300
   TCGATTCGCG GTATGGATTG GCTTCGGCTG AACAGACGCT TATTGGGTGC TTTTCCTGCT    360
   GTGGCTGGAT GGACTGGTGA ACCGTAGCTG TGCTAGGCTT GGCGTTTGAA CCGGCGGTGT    420
   GGTGCGAAGT AGGGTGTCTG TTCNGGCGCA AGCTGGGGTG GACGAGGGTC GATCCATTTG    480
   GGAAACGTTG TGNNNTTCGG CGCGCATCTC AATTGGACCT GATATCA                  527
(2) INFORMATION FOR SEQ ID NO: 27:
   (i) SEQUENCE CHARACTERISTICS:
      (A) LENGTH: 485 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora citricola
      (C) INDIVIDUAL ISOLATE: CIT1
   (viii) POSITION IN GENOME:
      (B) MAP POSITION: ITS2
   (xi) SEQUENCE DESCRIPTION: SEQ ID NO: 27:
   GGATTCAGTG AGTCATCGAA ATTTTGAACG CATATTGCAC TTCCGGGTTA GTCCTGGGAG    60
   TATGCCTGTA TCAGTGTCCG TACATCAAAC TTGGCTTTCT TCCTTCCGTG TAGTCGGTGG   120
   AGGATGTGCC AGATGTGAAG TGTCTTGCGG GTGTCCTTCG GGTCGTCTGC GAGTCCTTTG   180
   AAATGTACTG AACTGTACTT CTCTTTGCTC GAAAAGCGTG GTGTTGCTGG TTGTGGAGGC   240
   TGCCTGCGTG GCCAGTCGGC GACCGGTTTG TCTGCTGCAG CGTTTAATGG AGGAGTGTTC   300
   GATTCGCGGT ATGGTTGGCT TCGGCTGAAC AGGCGCTTAT TGTATGCTTT TCCTGCTGTG   360
   GCGTGATGGG CTGGTGAACC GTAGCTATGT GTGGCTTGGC TTTTGAATCG GCTTTGCTGT   420
   TGCGAAGTAG AGTGGCGGCT TCGGCTGTCG AGGGTCGATC CATTTGGGAA CTTTGTGNAC   480
   CTCGG                                                               485
(2) INFORMATION FOR SEQ ID NO: 28:
   (i) SEQUENCE CHARACTERISTICS:
      (A) LENGTH: 515 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora nicotianae
      (C) INDIVIDUAL ISOLATE: NIC1
   (viii) POSITION IN GENOME:
      (B) MAP POSITION: ITS2
   (xi) SEQUENCE DESCRIPTION: SEQ ID NO: 28:
   AGGATTCAGT GAGTCATCGA AATTTTGAAC GCATATTGCA CTTCCGGGTT AGTCCTGGAA    60
   GTATGCCTGT ATCAGTGTCC GTACATTAAA CTTGACTTTC TTCCTTCCGT GTAGTCGGTG   120
   GAGGAGATGT CAGATGTGAA GTGTCTTGCG ATTGGTCTTC GGACCGGCTG CGAGTCCTTT   180
   TAAATGTACT AAACTGAACT TCTCTTTGCT CGAAAAGTGG TGGCGTTGCT GGTTGTGAAG   240
   GCTGCTATTG TGGCAAATTG GCGACTGGTT TGTCTGCTGC GGCNTTAATG GAAGAGTGTT     300
   CGATTCGTGG TATGGTTGGC TTCGGCTGAA CAATGCAACT TATTGGACGT TTTTCCTGCT     360
   GTGGCGTGAT GGACTGGTGA ACCATAGCTC GGTGGCTTGG CTTTTGAATT GGCTTTGCTG     420
   TTGCGAAGTA GGGTGGCAGC TTCGGTTGTC GAGGGTCGAT CCATTTGGGA ACTTAATGTG     480
   TACTTCGGTA TGCATCTCAA TTGGACCTGA TATCC                               515
(2) INFORMATION FOR SEQ ID NO: 29:
   (i) SEQUENCE CHARACTERISTICS:
      (A) LENGTH: 512 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cactorum
      (C) INDIVIDUAL ISOLATE: CAC1
   (viii) POSITION IN GENOME:
      (B) MAP POSITION: ITS2
   (xi) SEQUENCE DESCRIPTION: SEQ ID NO: 29:
   GGATTCAGTG AGTCATCGAA ATTTTGAACG CATATTGCAC TTCCGGGTTA GTCCTGGGAG      60
   TATGCCTGTA TCAGTGTCCG TACATCAAAC TTGGCTTTCT TCCTTCCGTG TAGTCGGTGG     120
   AGGAGATGCC AGATGTGAAG TGTCTTGCGG CTGGTTTTCG GACCGACTGC GAGTCCTTTT     180
   AAATGTACTG AACTGTACTT CTCTTTGCTC GAAAAGCGTG GCGTTGCTGG TTGTGGAGGC     240
   TGCTATTGTA GCAAGTTGGC GACCGGTTTG TCTGCTGCGG CGTTAATGGA AGAGTGTTCG     300
   ATTCGCGGTA TGGTTAGCTT CGGCTGAACA ATGCGCTTAT TGGATGTTTT TTCTGCTGTG     360
   GCGTGATGGA CCGGTGAACC ATAGCTCAGT TGCTTGGCTT TTGAATCGGC TTTGCTNTTG     420
   CGAAGTAGAG TGGCGGCTTC GGCTGTCGAG GGTCGATCCA TTTGGGAAAT GTGTGTGTAC     480
   TTCGGTATGC ATCTCAATTG GACCTGATAT CA                                  512
(2) INFORMATION FOR SEQ ID NO: 30:
   (i) SEQUENCE CHARACTERISTICS:
      (A) LENGTH: 507 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora pseudotsugae
      (C) INDIVIDUAL ISOLATE: PSE1
   (viii) POSITION IN GENOME:
      (B) MAP POSITION: ITS2
   (xi) SEQUENCE DESCRIPTION: SEQ ID NO: 30:
   GCAGGATTCA GTGAGTCATC GAAATTTTGA ACGCATATTG CACTTCCGGG TTAGTCCTGG    60
   GAGTATGCCT GTATCAGTGT CCGTACATCA AACTTGGCTT TCTTCCTTCC GTGTAGTCGG   120
   TGGAGGAGAT GCCAGATGTG AAGTGTCTTG CGGCTGGTTT TCGGACCGAC TGCGAGTCCT   180
   TTTAAATGTA CTGAACTGTA CTTCTCTTTG CTCGAAAAGC GTGGCGTTGC TGGTTGTGGA   240
   GGCTGCTATT GTAGCAAGTT GGCGACCGGT TTGTCTGCTG CGGCGTTAAT GGAAGAGTGT   300
   TCGATTCGCG GTATGGTTGG CTTCGGCTGA ACAATGCGCT TATTGGATGA TTTTTCTGCT   360
   GTGGCGTGAT GGACCGGTGA ACCATAGCTC AGTGGCTTGG CTTTTGAATC GGCTTTGCTG   420
   TTGCGAAGTA GAGTGGCGGC TTCGGCTGTC GAGGGTCGAT TCATTTGGGA AATGTGTGTG   480
   TACTTCGGTA TGCATCTCAA TTGGACC                                       507
(2) INFORMATION FOR SEQ ID NO: 31:
   (i) SEQUENCE CHARACTERISTICS:
      (A) LENGTH: 507 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora idaei
      (C) INDIVIDUAL ISOLATE: IDA4
   (viii) POSITION IN GENOME:
      (B) MAP POSITION: ITS2
   (xi) SEQUENCE DESCRIPTION: SEQ ID NO: 31:
   GGATTNNGTG AGNCATCGAA ATTTTGAACG CATATTGCAC TTCCGGGTTA GTCCTGGAAG    60
   TATGCCTGTA TCAGTGTCCG TACATCAAAC TTGGCTTTCT TCCTTCCGTG TAGTCGGTGG   120
   AGGAGATGCC AGATGTGAAG TGTCTTGCGG CTGGTTTTCG GACCGACTGC GAGTCCTTTT   180
   AAATGTACTG AACTGTACTT CTCTTTGCTC GAAAAGCGTG GCGTTGCTGG TTGTGGAGGC   240
   TGCTATTGTA GCAAGTTGGT GACCGGTTTG TCTGCTGCGG CGTTAATGGA AGAGTGTTCG   300
   ATTCGCGGTA TGGTTGGCTT CGGCTGAACA ATGCGCTTAT TGGATGTTTT TTCTGCTGTG   360
   GCGTGATGGA CCGGTGAACC ATAGCTCAGT GGCTTGGCTT TTGAATCGGC TTTGCTGTTG   420
   CGGAGTAGAG TGGCGGCTTC GGCTGTCGAG GGTCGATCCA TTTGGGAAAT GTGTGTGTAC   480
   TTCGGTATGC ATCTCAATTG GACCTGA                                       507
(2) INFORMATION FOR SEQ ID NO: 32:
   (i) SEQUENCE CHARACTERISTICS:
      (A) LENGTH: 450 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora capsici
      (C) INDIVIDUAL ISOLATE: R255
   (viii) POSITION IN GENOME:
      (B) MAP POSITION: rDNA containing ITS2
   (ix) FEATURE:
      (A) NAME/KEY: misc_feature
      (B) LOCATION:21..443
      (D) OTHER INFORMATION:/standard_name= "ITS2 region"
   (xi) SEQUENCE DESCRIPTION: SEQ ID NO: 32:
   GWATGCCCTG TATCAGTGTC CGTACATCAA ACTTGGATTT CTTCCTTCCG ATGTAGTCGG    60
   TGGAGGATGT CCCAGATGTG AAGTGTCTTG MGTGTTGWCC WTCGGGTCGA CTGCGAGTCC   120
   TTTTAAATGT ACTGAACTGT ACTTCTCTTT GCTCGAAAAG CGTGGTGTTG CTGGTTGTGG   180
   AGGCTGCCTG CGTGGCCAGT CGGCGACCGG TTTGTCTGCT GCGGCGTTTA AAGGAGGAGT   240
   GTTCGATTCG CGGTATGGTT GGCWTCGGCT GAACAGACGC TTATTGAATG CWTWTCCTNC   300
   TGTGGCGTGA TGGGCTGGTG AACCGTAGCT GTGTTTGGCT TGGCTTATGA ATCGGCTTTG   360
   CTGTTNCGAA GTAGGGTGAC GGCTTCGGCT GTCGAGGGTC GATCCATTTT GGGAACTTTG   420
   TGTRCACKTC GGTGCGCATC TCAATTGGAC                                     450
(2) INFORMATION FOR SEQ ID NO: 33:
   (i) SEQUENCE CHARACTERISTICS:
      (A) LENGTH: 562 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora citricola
      (C) INDIVIDUAL ISOLATE: P812
   (viii) POSITION IN GENOME:
      (B) MAP POSITION: rDNA containing ITS2 region
   (ix) FEATURE:
      (A) NAME/KEY: misc_feature
      (B) LOCATION:143..560
      (D) OTHER INFORMATION:/standard_name= "ITS2 region"
   (xi) SEQUENCE DESCRIPTION: SEQ ID NO: 33:
   GTCTAGGCTC GCAACATCGA TGAAGAACGC TGCGAACTGC GATACGTAAT GCGAATTGCA 60
   GGATTCAGTG AGTCATCGAA ATTTTGAACG CATATTGCAC TTCCGGGTTA GTCCTGGGAG 120
   TATGCCTGTA TCAGTGTCCG TACATCAAAC TTGGCTTTCT TCCTTCCGTG TAGTCGGTGG 180
   AGGATGTGCC AGATGTGAAG TGTCTTGCGG GTGTCCTTCG GGTCGTCTGC GAGTCCTTTG 240
   AAATGTACTG AACTGTACTT CTCTTTGCTC GAAAAGCGTG GTGTTGCTGG TTGTGGAGGC 300
   TGCCTGCGTG GCCAGTCGGC GACCGGTTTG TCTGCTGCGG CGTTTAATGG AGGAGTGTTC 360
   GATTCGCGGT ATGGTTGGCT TCGGCTGAAC AGGCGCTTAT TGTATGCTTT TCCTGCTGTG 420
   GCGTGATGGG CTGGTGAACC GTAGCTGTGT GTGGCTTGGC TTTTGAATCG GCTTTGCTGT 480
   TGCGAAGTAG AGTGGCGGCT TCGGCTGTCG AGGGTCGATC CANTTGGGAA CTTTGTGTGC 540
   ACCTGGNCCG CATCTCAATT GG 562
(2) INFORMATION FOR SEQ ID NO: 34:
   (i) SEQUENCE CHARACTERISTICS:
      (A) LENGTH: 572 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora cryptogea
      (C) INDIVIDUAL ISOLATE: 252
   (viii) POSITION IN GENOME:
      (B) MAP POSITION: rDNA containing ITS2 region
   (ix) FEATURE:
      (A) NAME/KEY: misc_feature
      (B) LOCATION:101..530
      (D) OTHER INFORMATION:/standard_name= "ITS2 region"
   (xi) SEQUENCE DESCRIPTION: SEQ ID NO: 34:
   CGATACGTAA TGCGAATTGC AGGATTCAGT GAGTCATCGA AATTTTGACG CATATTGCAC 60
   TTCGGTTAGT CCTGGGAGTA TGCCTGTATC AGTGTCCGTA CACTAAACTT GCTCCCTTCC 120
   TTCCGTGTAG TCGGTGGATG GGGACGCGCA GATGTGAAGT GTCTTGCGGC TGGTCTTCGG 180
   TCCGCCTGCG AGTCCTTTGA AATGTACTAC ACTGAACTTC TCTTTGCTCG AAAAGCGTGA 240
   CGTTGCTGGT TGTGGAGCCT GCCTGTGTGG CATGTCGGCG ACCGGTTTGT CTGCTGCGGC 300
   GTTTAATGGA GGAGTGTTCG ATTCGCGGTA TGGTTGGCTT CGGCTGAACA GACGCTTATT 360
   GGGTGCTTTT CCTGCTGTGG CTGGATGGAC TGGTGAACCG TAGCTGTGCT AGGCTTGGCG 420
   TTTGAACCGG CGGTGTGGTG CGAAGTAGGG TGTCTGTTCT GGCGTAAGCT GGGGTGGACG 480
   AGGGTCGAGC CATTTGGGAA ACGTTGTGTG CGCTTCGAAA CGCATCTCTA TTGGGACACA 540
   TATCAGTCGA AGGGACCAAT CGACAAATTA GC 572
(2) INFORMATION FOR SEQ ID NO: 35:
   (i) SEQUENCE CHARACTERISTICS:
      (A) LENGTH: 600 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophthora drechslerii
      (C) INDIVIDUAL ISOLATE: P538
   (viii) POSITION IN GENOME:
      (B) MAP POSITION: rDNA containing ITS2 region
   (ix) FEATURE:
      (A) NAME/KEY: misc_feature
      (B) LOCATION:153..583
      (D) OTHER INFORMATION:/standard_name= "ITS2 region"
   (xi) SEQUENCE DESCRIPTION: SEQ ID NO: 35:
   AGTGGATGTC CTAGGCTCGC ACATCGATGA AGAACCCTGC GAACTGCGAA TACGTAATGC 60
   GAATTGCAGG ATTCAGTGAG TCATCGAAAT TTTGAACGCA TATTTGCACT TCCGGGATAA 120
   GTCCTGGGAG TATGCCTGTA TCAGTGTCCG AACACTAAAC TTGACTCCCT TCCTTCCGTG 180
   TAGTCGGTGG ATGGGGACGC ACAGATGTGA AGTGTCTTGC GNCTGGTCTT CGGTCCGGCT   240
   ACGAGTCCTA TTAAATGTAC TACACTGTAC TTCTCTTTGC TCGAAAAGCG TGACGTTGCT   300
   GGTTGTGGAG ACTCCCTGTG TGGCATGTCG GCGACCGGTT TGTCTSCTGC GGCGTTTTAA   360
   TGGAGGAGTG TTCGATTCGC GGTATGGTTG GCTTCGGCTG AACAGACGCT TATTGGGTGC   420
   TTTTTCTGCT GCGGCGGGAT GGACTGGTGA ACCGTAGCTG TGCTAGGCTT GGCGTTTGAA   480
   CCGGCGGTAT TGTGCGAAGT AGGGTGTCTG TCTCGGCGCA AGCTGGGGTG GGCGAGGGTC   540
   GATCCATTTG GGAAACGTTG TGTGCGCTTC GGCGTGCATC TCAATGGACC TGATATCAGC   600
(2) INFORMATION FOR SEQ ID NO: 36:
   (i) SEQUENCE CHARACTERISTICS:
      (A) LENGTH: 536 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Phytophtora gonapodyides
      (C) INDIVIDUAL ISOLATE: P245
   (viii) POSITION IN GENOME:
      (B) MAP POSITION: rDNA containing ITS2 region
   (ix) FEATURE:
      (A) NAME/KEY: misc_feature
      (B) LOCATION:91..519
      (D) OTHER INFORMATION:/standard_name= "ITS2 region"
   (xi) SEQUENCE DESCRIPTION: SEQ ID NO: 36:
   GAATTGCAGG ATTCAGTGAG TCATCGAAAT TTTGAACGCA TATTGCACTT CCGGGTTAGT    60
   CCTGGGAGTA TGCCTGTATC AGTGTCCGTA CATCAACCTT GGTTTTCTTC CTTCCGTGGA   120
   GTCGGTGGAG GAGATGCCAG ACGTGAAGTG TCTTGCTGGC GGTCTTTCGA GTCTGCCGGT   180
   GAGTCCTTTG AAATGTACTG AACTGTACTC TCTCTTTGCT CGAAAAGCGT GGCGTTGCTG   240
   GTTGTGGAGC CTGCCCGTGT GGCCAGTCGG CGACCGGTTC GTTAGCTGTG GCGTTTAATG 300
   GAGGAGTGTT CGATTCGCGG TATGGTTGGC TTCGGCTGAA CAATCTGCTT ATTGGGTGCT 360
   TTTCCTSCTG CAGCGGTATG AACTGGTGAA CCGTAGCTGT GTGGTGCTTG GCTTTTGAAC 420
   CGGCTTTGCT TTGCGAAGTA GAGTGGCGGC TTCGGCTGTC GAGGGGTCGA TCCATTTTGG 480
   GAAACTTTGT GTGTGCGGCT TCGTGCTGCG CGCATCTCAA TGGACCTGAT ATCAGC 536
(2) INFORMATION FOR SEQ ID NO: 37:
   (i) SEQUENCE CHARACTERISTICS:
      (A) LENGTH: 581 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Peronospora cristata
   (viii) POSITION IN GENOME:
      (B) MAP POSITION: rDNA containing ITS2 region
   (ix) FEATURE:
      (A) NAME/KEY: misc_feature
      (B) LOCATION:161..580
      (D) OTHER INFORMATION:/standard_name= "ITS2 region"
   (xi) SEQUENCE DESCRIPTION: SEQ ID NO: 37:
   CAACTTTCAG CAGTGGATGT CTAGGCTCGC ACATCGATGA AGAACGCTGC GAACTCGAAT 60
   ACGTAATGCG AATTTGCAGG ATTCAGTGAG TCATCGAAAT TTTGAACGCA TATTGCACTT 120
   CCGGGTTATC CCTGGGAGTA TGCCCTGTAT CAGTGTCCGT ACATCAAACT TGGTTTTCTT 180
   CTTTCCGTGT AGTCGGTGGA GGATATGCCA GATGTGAAGT GTCTTGCGGC TGGTTTTTGG 240
   ATCGSCTGTG AGTCCTTTGA AATGTACAGA ACTGACTCTC TTTGTTTGAA AAGCGTGGCG 300
   TTGCTGGTTG TGGAGGCTGT CAGTATGGCT AGTCGGCGAC CGGTTTGTCT GCTATGGCAT 360
   CATGGAGGAG TGTTCGATTC GCGGTATGGT TGGCTTCGGC TAAACAGGCG CTTATTGGAC 420
   GTTCTTTCTG CTATGGCGGT ATGAACTGGT GAACCGTAGT TCATGCATGA CTTGGCTTTT 480
   GAATCGGCTT TGCTGTGCGA AGTAGAGTGA CAGTTTCGGC TGTCGAGGGW CGACCCATTT 540
   GGGAACTTGT GCTGTGCGAC TTCGGTTGCG TGTCATCTCA A 581
(2) INFORMATION FOR SEQ ID NO: 38:
   (i) SEQUENCE CHARACTERISTICS:
      (A) LENGTH: 633 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Peronospora sparsa
   (viii) POSITION IN GENOME:
      (B) MAP POSITION: rDNA containing ITS2 region
   (ix) FEATURE:
      (A) NAME/KEY: misc_feature
      (B) LOCATION:211..633
      (D) OTHER INFORMATION:/standard_name= "ITS2 region"
   (xi) SEQUENCE DESCRIPTION: SEQ ID NO: 38:
   TAATTACTGA TTATACTGTG GGGACGAAAG TCTCTGCTTT TAACTAGATA GCAACTTTCA 60
   GCAGTGGATG TCTAGGCTCG CACATCGATG AAGAACGCTG CGAACTGCGA TACGTAATGC 120
   GAATTGCAGG ATTCAGTGAG TCATCGAAAT TTTGAACGCA TATTGCACTT CCGGGTTAGT 180
   CCTGGAAGTA TGCCTGTATC AGTGTCCGTA CATCAAACTT GGTTTTCTTC TTTCCGTGTA 240
   GTCGGTGGAG GATATGCCAG ATGTGAAGTG TCTTGCGGCT GATTTTCGGA TCGGCTGCGA 300
   GTCCTTTGAA ATGTACAGAA CTGTACTTCT CTTTGCTCGA AAAGCATGGC GTTGCTGATT 360
   GTGGAGGCTG TCCGCGTGNC CAGTCGGCGA TCGGTTTGTC TGCTGTGGCT TAATGGAGGA 420
   GTGTTTGATT CGCGGTATGA TTGGCTTCGG CTGAACAGGC GCTTATTGGA CGTTCTTTCT 480
   GCTATGGCGG TATGGACTGA TGAACCGTAG CTATGCGTTG ACTTGGCTTT TGAATTGGCT 540
   TTGCTGTTGC GAAGTAGAGT GGCAGTTTCA GCTGTCGAGG GTCGACCCAT ATTGGGAAAT 600
   TGTGCTGTGC GGCTTCGGTC GTGTGTCATC TCA 633
(2) INFORMATION FOR SEQ ID NO: 39:
   (i) SEQUENCE CHARACTERISTICS:
      (A) LENGTH: 636 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Peronospora viciae
   (viii) POSITION IN GENOME:
      (B) MAP POSITION: rDNA containing ITS2 region
   (ix) FEATURE:
      (A) NAME/KEY: misc_feature
      (B) LOCATION:191..619
      (D) OTHER INFORMATION:/standard_name= "ITS2 region"
   (xi) SEQUENCE DESCRIPTION: SEQ ID NO: 39:
   CCTGTGGGGA CGAAATCTCT GCTTTTAACA AGATAGCAAC TTTCAGCAGT GGATGTCCTA 60
   GGCTCGCACA TCGATGAAGA ACCCTCCGAC CTGCGATACG TAATGCGAAT TGCAGGATTC 120
   AGTGAGTCAT CGAAATTTTG AACGCATATT GCACAACCGG GGACCATCCC GGGAGTATGC 180
   CTGTATCAGT GTCCGTACAT CAAACTTGGT TTTCTTCTTT CCGTGTAGTC GGTGGAAGAT 240
   ATGCCAGATG TGAAGTGTCT TTCGACTGGT TTTCGAATCG GTTGTGAGTC CTTTGAAATG 300
   WATAGAACTG TACTTCTCTT TGCTCGAAAA GCGTGRCATT GTTGGTTGTG GAGCCTGTTC 360
   GTGTGACCAG TCGGCGATCG GTTTGTCTSC TGTAGCATTA ATGGAGGAGT GTTCGATTCG 420
   CGGTATGGTT GGCTTCGGCT AAACAGACGC TTATTGGGCT CTCTTTCTGC TGTGGCGGTA 480
   TGAACTGGTG AACCGTAGTC ATGTGTGACT TGGCTTTTGA ATTGGCTTTG CTGGTGCGAA 540
   GTAGAGTGAC AGTTTCGGCT GTCGAGGGTC GACCCATTTG GGAAATTTAT GCGGTGCGGC 600
   TTCGTCGCGC AGWAWCTCAA TTGGACCTGA TATCAG 636